# EUROPEAN PATENT APPLICATION

(11) **EP 1 892 530 A1**
(43) Date of publication of application: **27.02.2008**
(21) Application number: 06119511.1
(22) Date of filing: 25.08.2006
(51) Int. Cl.: G01N 33/542, G01N 33/60

(54) **Method for determining transport activity of a transport protein**

(71) Applicant: Boehringer Ingelheim Pharma GmbH & Co. KG, 55216 Ingelheim am Rhein (DE)
(72) Inventor: Cui, Yunhai, 88400 Biberach (DE)
(74) Representative: Hammann, Heinz

(57) **Abstract**

The present invention relates to a method for determining transport activity of a transport protein and its use for the identification of compounds which can modulate said transport activity.

## Description

### Introduction

Transport proteins are membranous proteins which regulate the selective permeability of biological membranes such as cytoplasmic or subcellular membranes. Transport proteins permit either the passive movement of solutes across the membrane down their electrochemical gradients (passive transporters) or mediate an accumulation of solutes against their concentration gradients by consuming directly or indirectly the energy provided by hydrolysis of ATP (active transporters). One of the largest families of active transporters are the ATP-binding cassette transporters (ABC transporters) (Higgins, 1992;Holland and Holland, 2005). In man, ABC transporters play important roles in a large variety of physiological and pathophysiological processes. Mutations in genes encoding ABC transporters result in diverse genetic diseases including cystic fibrosis (Riordan et al., 1989), Tangier disease (Bodzioch et al., 1999;Brooks-Wilson et al., 1999;Young and Fielding, 1999), Dubin-Johnson syndrome (Kartenbeck et al., 1996;Mayer et al., 1995), pseudoxanthoma elasticum (Ringpfeil et al., 2000), and Stargardt disease (Allikmets, 1997). Besides their physiological functions, ABC transporters also play important roles in the process of absorption, disposition and elimination of drugs (Ayrton and Morgan, 2001 ;Borst and Elferink, 2002). Overexpression of ABC transporters in tumor cells is a major mechanism of their resistance to chemotherapeutic drugs (Gottesman et al., 2002). Therefore, investigations on interaction between drug and ABC transporters are indispensable for the successful development of drug candidates.

Because of the important function of transport proteins there is a need for substances which can modulate the activity of transport proteins. For identifying a said substance, there is a need for e.g. a high or an ultra high throughput compatible assay which is sensitive enough to determine whether a substance can modulate activity of transport proteins.

Presently, only costly and time consuming methods comprising several steps are known in the art for determining transport activity of a transport protein at all - which is the basis method for determining whether a substance can modulate transport protein activity. Such a method for determining transport protein activity known in the art is for example a rapid filtration method wherein transport activity of a transport protein is measured as follows:
- providing membrane vesicles prepared from tissues, cultured cells, or cells expressing a recombinant transport protein,
- incubating said vesicles for a given period of time with a radiolabel substrate under conditions which allow transport of the substrate,
- separati ng membrane vesicles from the extra-vesicular substrate by filtration through e.g. nitrocellulose or fibreglass membranes, and
- measuring e.g. increased radioactivity of separated vesicles wherein the amount of radioactivity directly correlates with the transport activity of the transport protein (Keppler et al., 1998).]

Besides the time-consuming separation step of said method a further disadvantage of such a method is its not suitability for high through put screening (HTS) campaigns and in particular for ultra high through put screening (uHTS) campaigns.

As outlined above uses of transport proteins for substance profiling in development of a pharmaceutical substance are becoming more and more of importance. Therefore, there is a strong need for providing a new and fast method for determining transport activity of a transport protein which is also suitable for use in HTS campaigns.

### Description Of The Invention

Although transport proteins and their applicability for profiling putative substances and therewith the need for methods for determining the activity of a respective transport protein are known for years until now only consuming methods for determining transport activity of a transport protein are known which requires several steps comprising a filtration step outside of the reaction tube wherein the labelled substrate has to be separated from reaction mixture prior to measuring the respective read-out.

The present invention, however, provides for the first time an "in one tube method" which allows a fast and waste sparing method for determining transport activity of a transport protein which additionally is suitable in HTS as well as in uHTS campaigns.

A basis for the present invention is the finding that a very C-terminus, i.e. 1 up to 25 amino acids of a transport protein can be marked e.g. with an Histidine tag (Hagmann et al., 1999) and/or targeted e.g with an antibody or antibody fragment without rendering its transport activity. Therewith the very C-terminus can be used as an anchor in an assay for determining the activity of a said transport protein.

A further basis is a well known homogeneous and generic assay technology called scintillation proximity assay (SPA) technology which goes back e.g. to 1979 (Hart and Greenwald, 1979 Mol. Immunology. Vol. 16, 265-267). SPA can be used to measure radioactivity via proximity of the radioactivity to a scintillant which can be stimulated to emit light that can be detected on standard scintillation counters.
When a radioactive atom decays it releases sub-atomic particles such as electrons. The distance these particles will travel through water is limited and is dependent upon the energy of the particle- SPA relies upon this limitation. For example, when a tritium [³H] atom decays it releases a β-particle. If the [³H] atom is within 1.5 µm of a suitable scintillant molecule, the energy of the β-particle will be sufficient to reach the scintillant and excite it to emit light. If the distance between the scintillant and the [³H] atom is greater than 1.5 µm, the β-particles will not have sufficient energy to travel the required distance. In an aqueous solution, collisions with water molecules dissipate the β-particle energy and it therefore cannot stimulate the scintillant.

Preferably, the scintillant is incorporated into small fluomicrospheres (beads) which are well known in the art. They are constructed to bind specific molecules (e.g. coated with Protein A to bind with an immunoglobulin). If a radioactive molecule is bound to the bead it is brought in close enough proximity that it can stimulate the scintillant to emit light (wavelength between 350 and 650 nm) which can be detected on standard scintillation counters like TopCount (PerkinElmer) or LEADSeeker (GE).

The present invention provides for the first time an SPA assay for determining the activity of a transport protein wherein the very C-terminus of a transport protein is used as a bridge to provide proximity to an SPA bead.

A method of the present invention for determining transport activity of a transport protein is characterized in that the method comprises,
- mixing of
   (i) a vesicle, preferably inside-out vesicle which harbours at least one transport protein in a way that the very C-terminus of the transport protein is outside of the vesicle with
   (ii) a SPA bead suitable for direct or indirect binding with the very C-terminus of the transport protein with
   (iii) - only in case of indirect binding of a SPA bead with the very C-terminus of the transport protein - at least one molecule which can mediate binding of the SPA bead with the very C-terminus of the transport protein with
   (iv) a radioactively marked substrate according to the invention which can be transported by the transport protein under condition which allow transport of the substrate, and
- incubating the mixture for a time period sufficiently long to enable direct or indirect binding of the vesicles with the SPA bead and to enable substrate transport into the vesicle, and
- measuring light emitted by scintillant of the bead.
   A further method of the present invention is a method for determining whether a compound is a modulator of transport activity of a transport protein characterized in that the method comprises,
- mixing of
   (A)
      (i) a vesicle, preferably inside-out vesicle which harbours at least one transport protein in a way that the very C-terminus of the transport protein is outside of the vesicle with
      (ii) a SPA bead suitable for direct or indirect binding with the very C-terminus of the transport protein with
      (iii) - only in case of indirect binding of a SPA bead with the very C-terminus of the transport protein - at least one molecule which can mediate binding of the SPA bead with the very C-terminus of the transport protein with
      (iv) a radioactively marked substrate according to the invention which can be transported by the transport protein under condition which allow transport of the substrate with
      (v) a compound to be tested, and mixing of
   (B)
      (i) a vesicle, preferably inside-out vesicle which harbours at least one transport protein in a way that the very C-terminus of the transport protein is outside of the vesicle with
      (ii) a SPA bead suitable for direct or indirect binding with the very C-terminus of the transport protein with
      (iii) - only in case of indirect binding of a SPA bead with the very C-terminus of the transport protein - at least one molecule which can mediate binding of the SPA bead with the very C-terminus of the transport protein with
      (iv) a radioactively marked substrate according to the invention which can be transported by the transport protein under condition which allow transport of the substrate, and
- incubating the mixture (A) and (B) for the same time period sufficiently long to enable direct or indirect binding of the vesicles with the SPA bead and to enable substrate transport into the vesicle, and
- measuring light emitted by scintillant of the bead, wherein increased value obtained with (A), when compared with (B), identifies a compound which is a activator of the transport protein tested and a decreased value obtained with (A), when compared with(B), identifies a compound which is an inhibitor of the transport protein tested.

All methods of the present invention can be used in a high throughput screening (HTS) as well as in an ultra HTS (uHTS) campaign. Such a method can be performed by using a plate having at least 96 wells, more preferred is the use of a 384 well plate or a 1536 well plate. Also preferred is the use of a chip as reaction and/or readout platform.

The present invention also provides for a kit for determining transport activity of a transport protein and for determining whether a substance is a modulator - i.e. an activator or an inhibitor - of a transport protein of the present invention.
A kit of the present invention for determining transport activity of a transport protein comprises,
(a) a vesicle, preferably an inside-out vesicle which harbours at least one transport protein in a way that the very C-terminus of the transport protein is outside of the vesicle,
(b) SPA bead suitable for direct or indirect binding with the very C-terminus of the transport protein.

The term "mixing" according to the methods of the present invention should be understood as at least any adding of ingredients mentioned. However, in the context of the present invention the term "mixing" can also include any movement or stirring which can lead to a further distribution of said ingredients.

A vesicle preferably an inside-out vesicle of the present invention is a vesicle which consists of a lipid bilayer membrane. An inside-out vesicle is a vesicle with its formerly intracellular surface now facing the outside of the vesicle and the formerly extracellular surface facing the inside out the vesicle. Such a vesicles is formed spontaneously during the preparation of cellular membranes. The sidedness of the membrane vesicles (rightside-out or inside-out) can be determined by method well known in the art, for example by measuring the activity of an ectoenzyme such as nucleotide pyrophsphatase (EC3.6.1.9), in the presence or absence of Triton X-1 00 (Keppler et al., 1998;Meier and Boyer, 1990).

A transport protein of the present invention is any protein naturally occurring or occurring due to recombinant expression in a cellular vesicle, cellular membrane or cellular cell-membrane which enables the transport of a substrate across the vesicle membrane as well as any such transport protein which has been rendered at the C-terminal end in a way that a further molecule can specifically bind to the rendered part of the C-terminus - e.g. a histidine-tag that has been fused to the C-terminus . A preferred transport protein is an ABC transport protein (Holland and Holland, 2005). A more preferred transport protein is selected from a group consisting of MRP1, MRP2, MRP3, MRP4, MRP5, MRP6, MRP7, MRP8, SUR1, SUR2, CFTR, ABCA1, ABCA3, ABCA4, ABCG5, ABCG8, MDR1, MDR3, BSEP, BCRP, TAP1, and TAP2 which are well known in the art. However, in the following References and Accession numbers thereof are mentioned:
Protein ID /Gene symbol; Reference; Accession Nr
MRP1 / ABCC1; (Cole et al., 1992); NP_004987
MRP2 / ABCC2; (Buchler et al., 1996); NP_000383
MRP3 / ABCC3; (Kiuchi et al., 1998); NP_003777
MRP4 / ABCC4; (Lee et al., 1998); NP_005836
MRP5 / ABCC5; (Belinsky et al., 1998); NP_005679
MRP6 / ABCC6; (Kool et al., 1999); NP_001162
CFTR / ABCC7; (Riordan et al., 1989); NP_000483
SUR1 / ABCC8; (Aguilar-Bryan et al., 1995); NP_000343
SU R2 / ABCC9; (Inagaki et al., 1996); NP_005682; NP_064693; NP_064694
MRP7 / ABCC10; (Hopper et al., 2001); NP_258261
MRP8 / ABCC11; (Tammur et al., 2001); NP_115972; NP_149163; NP_660187
MDR1 / ABCB1; (Gros et al., 1986); NP_000918
TAP1 / ABCB2; (Trowsdale et al., 1990); NP_000584
TAP2 / ABCB3; (Trowsdale et al., 1990); NP_000535; NP_061313
MDR3 / ABCB4; (Van der Bliek et al., 1987); NP_000434; NP_061337; NP_061338
BSEP/ABCB11; (Strautnieks et al., 1998); NP_003733
ABCA1; (Luciani et al., 1994); NP_005493
ABCA3; (Klugbauer and Hofmann, 1996); NP_001080
ABCA4; (Allikmets, 1997); NP_000341
BCRP / ABCG2; (Allikmets et al., 1998); NP_004818
ABCG5; (Berge et al., 2000); NP_071881
ABCG8; (Berge et al., 2000); NP_071882

A preferred transport protein is a protein capable of transporting a substrate according to the invention from the outside into a vesicle according to the invention.

A very C-terminus of a transport protein of the present invention is the very end of a transport protein according to the present invention, preferably comprising a stretch of at least 6 up to 25 amino acids of the very C-terminal end of a transport protein according to the present invention. Such a peptide consisting of amino acids of such a stretch can be used according to the present invention to generate antibodies or antibody fragments which can bind to a said peptide and therewith can bind to the very C-terminal end of a transport protein according to the present invention and can be used to mediate indirect binding of a SPA bead according to the invention with a very C-terminus according to the invention. As an example two peptides consisting of amino acids of a stretch of 15 and 25 amino acids of MRP4 are given in SEQ ID NO: 1 and 2, respectively.

An activity of a transport protein of the present invention is every activity of a transport protein according to the invention which results in a spatial alteration of a substrate according to the present invention, preferably results in a translocation of a substrate according to the invention into a vesicle according to the invention. Said activity can be determined via a method according to the invention.

A SPA-bead of the present invention is every SPA bead which can bind directly or indirectly to the very C-terminus of a transport protein according to the invention. An indirect binding of the invention is a binding which is mediated by a further soluble molecule which can be an antibody (first antibody) which can on the one hand bind to the very C-terminus of a transport protein according to the invention and on the other hand can bind with an SPA bead e.g. via Protein A (Protein A is a protein isolated from the cell wall of a number of strains of the bacteria *Staphylococcus aureus* and is characterized by its ability to bind to the IgG of most mammalian species.) or via a further antibody which has been coupled to the SPA bead and which is able to bind to the first antibody (e.g. an anti-rabbit, an anti-goat, an anti-rat, or an anti-mouse antibody which is capable of binding with immunoglobulins from rabbit, goat, rat, or mouse, respectively). In such a case the SPA bead preferably is coated with Protein A, or a respective second antibody.
A direct binding of the invention is a binding wherein no further soluble molecule is needed, for example such a binding can be a binding of the transport protein according to the present invention to a SPA bead which has been coated in a way that a binding with the very C-terminus or a rendered very C-terminus is possible, e.g. a transport protein according to the present invention to which C-terminus a histidine-tag has been fused binds directly with an SPA bead which has been coated with copper-chelate.
A suitable amount of SPA beads can easily be determined by methods commonly known in the art. However, amounts between 20-250 µg/well of a 384-well plate are preferably suitable.

An antibody which can bind with the C-terminus of a transport protein of the present invention is any antibody (Ab) of a polyclonal serum, e.g. rabbit serum, or any monoclonal antibody (mAb) which can bind with the C-terminus of a transport protein according to the present invention. Such an antibody of the invention can easily be obtained by methods know in the art, i.e. by immunization of a respective non-human animal by administration of at least a C-terminus of a transport protein of the invention. Whether a said Ab of a polyclonal serum or mAb can be used in a method of the invention can easily be tested by well know methods in the art which allow to determine whether a mAb or Ab of a polyclonal serum can bind - preferably can bind selectively - to the stretch of 15 up to 25 amino acids of the very C-terminal end of the transport protein of the invention. According to the present invention it should be understood that a suitable Ab or mAb of the present invention does not bind to a different amino acid domain which is (i) situated outside of a vesicle if integrated in the membrane of a said vesicle and which is (ii) upstream of said 25 amino acids of the C-terminus of a transport protein of the present invention.

A histidine-tag of the present invention is a peptide which consists of histidine residues preferably of at least 6 histidine residues. It is engineered to the very end of the transport protein according to the present invention by e.g. inserting a DNA sequence encoding the histidine residues in front of the stop codon of the full-length cDNA of the transport protein. Further methods for achieving a said tag are well known in the art.

A substrate of the present invention is any endogenous molecule or chemical compound or oligo-peptide or nucleic acid or nucleotide or cyclic nucleotide which can be transported by a transport protein according to the present invention. Such a transport of to the present invention is any spatial alteration of a substrate of the invention caused by a transport protein which usually is integrated in a lipid biolayer.

A preferred substrate is selected from a group consisting of: ADP, BQ-123, cholesterol, cimetidine, colchicine, cyclic AMP, cyclic GMP, dehydroepiandrosterone sulfate, daunomycin, digoxin, estradiol glucuronide, estrone 3-sulfate, etoposide, folic acid, glucosylceramide, glycochenodeoxycholate, glycocholate, leukotriene C4, leukotriene D4, leukotriene E4, methotrexate, mitoxanthone, sulfobromophthalein, paclitaxel, platelet activating factor, prostaglandin E1, prostaglandin E2, prostaglandin F2alpha, ritonavir, saquinavir thromboxane B2, verapamil, sitosterol, taurochenodeoxycholate, taurocholate, tauroursodeoxycholate, prazosin, vincristine and vinblastine. More preferred combinations of a transport protein and a substrate are as follows:

| protein ID / gene symbol with substrate (References) | |
|---|---|
| MRP1/ABCC1 : | leukotriene C4, leukotriene D4, leukotriene E4, estradiol glucuronide, folic acid, or methotrexate (Jedlitschky et al., 1996; Keppler et al., 1998; Leier et al., 1994) |
| MRP2 / ABCC2 : | leukotriene C4, estradiol glucuronide, folic acid, methotrexate, or sulfobromophthalein (Bakos et al., 2000; Cui et al., 1999; Cui et al., 2001; Hooijberg et al., 1999; Hooijberg et al., 2003) |
| MRP3 / ABCC3 : | leukotriene C4, estradiol glucuronide, folic acid, methotrexate, or glycocholate (Hooijberg et al., 2003; Zeng et al., 2000) |
| MRP4 / ABCC4 : | leukotriene C4, estradiol glucuronide, estrone 3-sulfate, dehydroepiandrosterone sulphate, folic acid, cGMP, cAMP, ADP, methotrexate, prostaglandin E1, prostaglandin E2, prostaglandin F2alpha, or thromboxane B2 (Chen et al., 2001; Chen et al., 2002; Jedlitschky et al., 2004;Reid et al., 2003; Rius et al., 2003;Rius et al., 2005; van Aubel et al., 2002; Zelcer et al., 2003) |
| MRP5 / ABCC5 : | folic acid, cGMP, cAMP, methotrexate, 5-fluoro-2'-deoxyuridine 5'-monophosphate, 5-fluoro-uridine 5'-monophosphate, or 2'-deoxyuridine 5'-onophosphate (Pratt et al., 2005; Wielinga et al., 2005) |
| MRP6 / ABCC6 : | leukotriene C4, or BQ-123 (llias et al., 2002; Madon et al., 2000) |
| CFTR / ABCC7 : | Chloride (Berger et al., 1991) |
| MRP7/ABCC10 : | leukotriene C4, estradiol glucuronide (Chen et al., 2003a) |
| MRP8 / ABCC11 : | cAMP, cGMP, leukotriene C4, estradiol lucuronide, estrone 3-sulfate, dehydroepiandrosterone sulphate, taurocholate, or glycocholate (Bortfeld et al., 2006; Chen et al., 2005) |
| MDR1/ABCB1 : | glucosylceramide, platelet activating factor, daunomycin, digoxin, colchicine, etoposide, paclitaxel, verapamil, vincristine, vinblastine, ritonavir, or saquinavir (Raggers et al., 1999; Raggers et al., 2001; Sarkadi et al., 1992;Takeuchi et al., 2006; Tanigawara et al., 1992) |
| BSEP / ABCB11 : | tauroholate, glycocholate, taurochenodeoxycholate, glycochenodeoxycholate, or tauroursodeoxycholate (Byrne et al., 2002; Noe et al., 2002) |
| ABCA4 : | retinal (Sun et al., 1999) |
| BCRP / ABCG2 : | estrone 3-sulfate, estradiol glucuronide, folic acid, methotrexate, mitoxanthone, topotecan, or cimetidine (Chen et al., 2003b; Imai et al., 2003; Pavek et al., 2005; Volk and Schneider, 2003) |
| ABCG5 : | cholesterol, or sitosterol (Wang et al., 2006) |
| ABCG8 : | cholesterol, or sitosterol (Wang et al., 2006) |

The substrate of the invention is radioactively labelled. Suitable isotopes are H³, S³⁵, P^{32 or 33}, J¹²⁵, - said listing only disclose suitable isotopes and should in no way being understood as restricted. A suitable concentration of a radioactively labelled substrate can easily be determined by methods commonly known in the art. However, concentrations between 1 and 1000 µM are preferably suitable.

Incubation period of the present invention is at least 30 seconds. Preferably, an incubation period lasts at least for 30 seconds and lasts no longer than 30 hours. A more preferred incubation period lasts at least for 1 hour and lasts no longer than 24 hours. Preferably luminescence signals are immediately measured after the end of the incubation period which can easily be measured after 0.5, 1, 2, 4, 6 hours or up to 24 hours. A suitable incubation period for each transport protein according to the invention can easily be identified by a skilled artisan via measurement of luminescence signal at different point in times and subsequent comparison of data obtained. Preferred incubation periods are e.g. those which provide significant data within the shortest time period.

A compound to be tested of the present invention to be a modulator of a transport protein according to the present invention is any small chemical molecule, peptide, or antibody.

A modulator of a transport protein of the present invention does influence the transport activity of a transport protein according to the present invention. Such an modulator is either an inhibitor of a transport protein of the invention or an activator of a transport protein of the invention. A said inhibitor does reduce or inhibit the transport activity of a transport protein of the invention whereas a said activator does enhance the transport activity of a transport protein of the invention.

The present invention also disclose modulators of a transport protein according to the present invention which have been determined as an inhibitor using a method according to the present invention, such as, e.g. MK571 or Dipyridamole which are inhibitors of MRP4 /(van Aubel et al., 2002).

The following Examples are meant to illustrate the present invention, however, shall not be construed as limitation. However, the Examples describe most preferred embodiments of the invention.

### Examples:

### Example 1:

1) Membrane vesicles from insect cells (Hi5 or Sf9) infected with a baculovirus construct containing the full-length cDNA encoding MRP4 are prepared as described by van Aubel et al. (van Aubel et al., 2002) with some modifications: Hi5 cells are cultured in suspension to a cell density of 1 x 10⁶ cells / ml in Insect-Xpress serum free insect cell medium and infected with baculovirus construct containing the full-length cDNA encoding MRP4. Three days after infection, cells are collected by centrifugation at 3000 rpm for 10 min. Cell pellet is resuspended in hypotonic buffer (0.1 mM EDTA, 1 mM Tris, 1 mM HEPES, pH=7.4) containing a cocktail of protease inhibitors (e.g. Complete, Roche Diagnostics, Article-Nr 04693132001) and stirred on a ice bath for 90 min. The disrupted cells are precipitated by centrifugation at 4°C and 100000g for 30 min. The pellet consisting of crude membrane fractions is resuspended in Tris/sucrose buffer (10 mM Tris, 250 mM sucrose, pH=7.4) and homogenized by 30 strikes in a glass dounce with a tight-fitting pestle. Protein concentration of the membrane preparation is determined using standard methods, e.g. the BCA protein assay (Pierce, product number 23250).
2) Determination of transport activity of MRP4: Membrane vesicles prepared in 1) are diluted in Tris/sucrose buffer to a concentration of 3.3 µg protein / µl. 5 µl of the diluted membrane vesicles is added to each well of a 384-well white opaque microplate (PerkinElmer, product number 6007299). 5 µl of a test compound (diluted in Tris/sucrose buffer to different concentrations) is added to the membrane vesicles. A reaction mix containing 12 mM ATP, 30 mM MgCl₂, 30 mM phosphocreatineand 22.5 mM K₂CO₃, 0.375 mg/ml creatine kinase ((Leier et al., 1994), Roche diagnostics, article-Nr. 10127566001), 90 µM folic acid, 0.1 µCi ³H-folic acid (Movarek Biochemicals, MT783), 120 µg Protein A SPA imaging beads or Protein A SPA scintillation beads (Matsumura et al., 1992), and 0.075 µl anti-MRP4 rabbit antiserum (directed against the C-terminal 15 amino acids of MRP4 as given in SEQ ID NO: 1 :) in Tris/sucrose buffer is prepared. The transport is started by adding 5 µl reaction mix to the membrane vesicles. Luminescence signals are measured with the LEADSeeker or TopCount system after 30 min, 1 h, 2h, 4h, or up to 24 h.
3) Calculation of inhibitory effect of a test compound: The relative transport activities of MRP4 determined in the presence of a test compound is calculated as percentage of luminescence determined in the presence of the test compound compared to luminescence determined in the absence of the test compound. An inhibitor of MRP4 will result in values lower than 100%, an activator of MRP4 will result in values higher than 100%.

Table 1 summarises the results obtained by employing the assay described above and testing known MRP4 inhibitors.

| | **IC50 [µM]** |
|---|---|
| Dipyridamole (van Aubel et al., 2002) | 35 |
| MK571 (van Aubel et al., 2002) | 3.6 |

### Literature

1. Aguilar-Bryan,L., Nichols,C.G., Wechsler,S.W., Clement,J.P., Boyd,A.E., III, Gonzalez,G., Herrera-Sosa,H., Nguy,K., Bryan,J., and Nelson,D.A. (1995). Cloning of the beta cell high-affinity sulfonylurea receptor: a regulator of insulin secretion. Science 268, 423-426.
2. Allikmets,R. (1997). A photoreceptor cell-specific ATP-binding transporter gene (ABCR) is mutated in recessive Stargardt macular dystrophy. Nat. Genet. 17, 122.
3. Allikmets,R., SchrimI,L.M., Hutchinson,A., Romano-Spica,V., and Dean,M. (1998). A human placenta-specific ATP-binding cassette gene (ABCP) on chromosome 4q22 that is involved in multidrug resistance. Cancer Res. 58, 5337-5339.
4. Ayrton,A., and Morgan,P. (2001). Role of transport proteins in drug absorption, distribution and excretion. Xenobiotica 31, 469-497.
5. Bakos,E., Evers,R., Sinko,E., Varadi,A., Borst,P., and Sarkadi,B. (2000). Interactions of the human multidrug resistance proteins MRP1 and MRP2 with organic anions. Mol. Pharmacol. 57, 760-768.
6. Belinsky,M.G., Bain,L.J., Balsara,B.B., Testa,J.R., and Kruh,G.D. (1998). Characterization of MOAT-C and MOAT-D, new members of the MRP/cMOAT subfamily of transporter proteins. J. Natl. Cancer Inst. 90, 1735-1741.
7. Berge,K.E., Tian,H., Graf,G.A., Yu,L., Grishin,N.V., Schultz,J., Kwiterovich,P., Shan,B., Barnes,R., and Hobbs,H.H. (2000). Accumulation of dietary cholesterol in sitosterolemia caused by mutations in adjacent ABC transporters. Science 290, 1771-1775.
8. Berger,H.A., Anderson,M.P., Gregory,R.J., Thompson,S., Howard,P.W., Maurer,R.A., Mulligan,R., Smith,A.E., and WeIsh,M.J. (1991). Identification and regulation of the cystic fibrosis transmembrane conductance regulator-generated chloride channel. J. Clin. Invest 88, 1422-1431.
9. Bodzioch,M., Orso,E., Klucken,J., Langmann,T., Bottcher,A., Diederich,W., Drobnik,W., Barlage,S., Buchler,C., Porsch-Ozcurumez,M., Kaminski,W.E., Hahmann,H.W., Oette,K., Rothe,G., Aslanidis,C., Lackner,K.J., and Schmitz,G. (1999). The gene encoding ATP-binding cassette transporter 1 is mutated in Tangier disease. Nat. Genet. 22, 347-351.
10. Borst,P., and Elferink,R.O. (2002). Mammalian ABC transporters in health and disease. Annu. Rev. Biochem. 71, 537-592.
11. Bortfeld,M., Rius,M., Konig,J., Herold-Mende,C., Nies,A.T., and Keppler,D. (2006). Human multidrug resistance protein 8 (MRP8/ABCC11), an apical efflux pump for steroid sulfates, is an axonal protein of the CNS and peripheral nervous system. Neuroscience 137, 1247-1257.
12. Brooks-Wilson,A., Marcil,M., Clee,S.M., Zhang,L.H., Roomp,K., van Dam,M., Yu,L., Brewer,C., Collins,J.A., Molhuizen,H.O., Loubser,O., Ouelette,B.F., Fichter,K., Ashbourne-Excoffon,K.J., Sensen,C.W., Scherer,S., Mott,S., Denis,M., Martindale,D., Frohlich,J., Morgan,K., Koop,B., Pimstone,S., Kastelein,J.J., Genest,J., Jr., and Hayden,M.R. (1999). Mutations in ABC1 in Tangier disease and familial high-density lipoprotein deficiency. Nat. Genet. 22, 336-345.
13. Buchler,M., Konig,J., Brom,M., Kartenbeck,J., Spring,H., Horie,T., and Keppler,D. (1996). cDNA cloning of the hepatocyte canalicular isoform of the multidrug resistance protein, cMrp, reveals a novel conjugate export pump deficient in hyperbilirubinemic mutant rats. J. Biol. Chem. 271, 15091-15098.
14. Byrne,J.A., Strautnieks,S.S., Mieli-Vergani,G., Higgins,C.F., Linton,K.J., and Thompson,R.J. (2002). The human bile salt export pump: characterization of substrate specificity and identification of inhibitors. Gastroenterology 123, 1649-1658.
15. Chen,Z.S., Guo,Y., Belinsky,M.G., Kotova,E., and Kruh,G.D. (2005). Transport of bile acids, sulfated steroids, estradiol 17-beta-D-glucuronide, and leukotriene C4 by human multidrug resistance protein 8 (ABCC11). Mol. Pharmacol. 67, 545-557.
16. Chen,Z.S., Hopper-Borge,E., Belinsky,M.G., Shchaveleva,l., Kotova,E., and Kruh,G.D. (2003a). Characterization of the transport properties of human multidrug resistance protein 7 (MRP7, ABCC10). Mol. Pharmacol. 63, 351-358.
17. Chen,Z.S., Lee,K., and Kruh,G.D. (2001). Transport of cyclic nucleotides and estradiol 17-beta-D-glucuronide by multidrug resistance protein 4. Resistance to 6-mercaptopurine and 6-thioguanine. J Biol. Chem. 276, 33747-33754.
18. Chen,Z.S., Lee,K., Walther,S., Raftogianis,R.B., Kuwano,M., Zeng,H., and Kruh,G.D. (2002). Analysis of methotrexate and folate transport by multidrug resistance protein 4 (ABCC4): MRP4 is a component of the methotrexate efflux system. Cancer Res. 62, 3144-3150.
19. Chen,Z.S., Robey,R.W., Belinsky,M.G., Shchaveleva,l., Ren,X.Q., Sugimoto,Y., Ross,D.D., Bates,S.E., and Kruh,G.D. (2003b). Transport of methotrexate, methotrexate polyglutamates, and 17beta-estradiol 17-(beta-D-glucuronide) by ABCG2: effects of acquired mutations at R482 on methotrexate transport. Cancer Res. 63, 4048-4054.
20. Cole,S.P., Bhardwaj,G., Gerlach,J.H., Mackie,J.E., Grant,C.E., Almquist,K.C., Stewart,A.J., Kurz,E.U., Duncan,A.M., and Deeley,R.G. (1992). Overexpression of a transporter gene in a multidrug-resistant human lung cancer cell line. Science 258, 1650-1654.
21. Cui,Y., Konig,J., Buchholz,J.K., Spring,H., Leier,l., and Keppler,D. (1999). Drug resistance and ATP-dependent conjugate transport mediated by the apical multidrug resistance protein, MRP2, permanently expressed in human and canine cells. Mol. Pharmacol. 55, 929-937.
22. Cui,Y., Konig,J., and Keppler,D. (2001). Vectorial transport by double-transfected cells expressing the human uptake transporter SLC21 A8 and the apical export pump ABCC2. Mol. Pharmacol. 60, 934-943.
23. Gottesman,M.M., Fojo,T., and Bates,S.E. (2002). Multidrug resistance in cancer: role of ATP-dependent transporters. Nat. Rev. Cancer 2, 48-58.
24. Gros,P., Ben Neriah,Y.B., Croop,J.M., and Housman,D.E. (1986). Isolation and expression of a complementary DNA that confers multidrug resistance. Nature 323, 728-731.
25. Hagmann,W., Nies,A.T., Konig,J., Frey,M., Zentgraf,H., and Keppler,D. (1999). Purification of the human apical conjugate export pump MRP2 reconstitution and functional characterization as substrate-stimulated ATPase. Eur. J. Biochem. 265, 281-289.
26. Higgins,C.F. (1992). ABC transporters: from microorganisms to man. Annu. Rev. Cell Biol. 8, 67-113.
27. Holland,K.A., and Holland,I.B. (2005). Adventures with ABC-proteins: highly conserved ATP-dependent transporters. Acta Microbiol. Immunol. Hung. 52, 309-322.
28. Hooijberg,J.H., Broxterman,H.J., Kool,M., Assaraf,Y.G., Peters,G.J., Noordhuis,P., Scheper,R.J., Borst,P., Pinedo,H.M., and Jansen,G. (1999). Antifolate resistance mediated by the multidrug resistance proteins MRP1 and MRP2. Cancer Res. 59, 2532-2535.
29. Hooijberg,J.H., Peters,G.J., Assaraf,Y.G., Kathmann,l., Priest,D.G., Bunni,M.A., Veerman,A.J., Scheffer,G.L., Kaspers,G.J., and Jansen,G. (2003). The role of multidrug resistance proteins MRP1, MRP2 and MRP3 in cellular folate homeostasis. Biochem. Pharmacol. 65, 765-771.
30. Hopper,E., Belinsky,M.G., Zeng,H., Tosolini,A., Testa,J.R., and Kruh,G.D. (2001). Analysis of the structure and expression pattern of MRP7 (ABCC10), a new member of the MRP subfamily. Cancer Lett. 162, 181-191.
31. Ilias,A., Urban,Z., SeidI,T.L., Le Saux,O., Sinko,E., Boyd,C.D., Sarkadi,B., and Varadi,A. (2002). Loss of ATP-dependent transport activity in pseudoxanthoma elasticum-associated mutants of human ABCC6 (MRP6). J. Biol. Chem. 277, 16860-16867.
32. Imai,Y., Asada,S., Tsukahara,S., Ishikawa,E., Tsuruo,T., and Sugimoto,Y. (2003). Breast cancer resistance protein exports sulfated estrogens but not free estrogens. Mol. Pharmacol. 64, 610-618.
33. Inagaki,N., Gonoi,T., Clement,J.P., Wang,C.Z., Aguilar-Bryan,L., Bryan,J., and Seino,S. (1996). A family of sulfonylurea receptors determines the pharmacological properties of ATP-sensitive K+ channels. Neuron 16, 1011-1017.
34. Jedlitschky,G., Leier,l., Buchholz,U., Barnouin,K., Kurz,G., and Keppler,D. (1996). Transport of glutathione, glucuronate, and sulfate conjugates by the MRP gene-encoded conjugate export pump. Cancer Res. 56, 988-994.
35. Jedlitschky,G., Tirschmann,K., Lubenow,L.E., Nieuwenhuis,H.K., Akkerman,J.W., Greinacher,A., and Kroemer,H.K. (2004). The nucleotide transporter MRP4 (ABCC4) is highly expressed in human platelets and present in dense granules, indicating a role in mediator storage. Blood 104, 3603-3610.
36. Kartenbeck,J., Leuschner,U., Mayer,R., and Keppler,D. (1996). Absence of the canalicular isoform of the MRP gene-encoded conjugate export pump from the hepatocytes in Dubin-Johnson syndrome. Hepatology 23, 1061-1066.
37. Keppler,D., Jedlitschky,G., and Leier,l. (1998). Transport function and substrate specificity of multidrug resistance protein. Methods Enzymol. 292, 607-616.
38. Kiuchi,Y., Suzuki,H., Hirohashi,T., Tyson,C.A., and Sugiyama,Y. (1998). cDNA cloning and inducible expression of human multidrug resistance associated protein 3 (MRP3). FEBS Lett. 433, 149-152.
39. Klugbauer,N., and Hofmann,F. (1996). Primary structure of a novel ABC transporter with a chromosomal localization on the band encoding the multidrug resistance-associated protein. FEBS Lett. 391, 61-65.
40. Kool,M., van der,L.M., de Haas,M., Baas,F., and Borst,P. (1999). Expression of human MRP6, a homologue of the multidrug resistance protein gene MRP1, in tissues and cancer cells. Cancer Res. 59, 175-182.
41. Lee,K., Belinsky,M.G., Bell,D.W., Testa,J.R., and Kruh,G.D. (1998). Isolation of MOAT-B, a widely expressed multidrug resistance-associated protein/canalicular multispecific organic anion transporter-related transporter. Cancer Res. 58, 2741-2747.
42. Leier,l., Jedlitschky,G., Buchholz,U., Cole,S.P., Deeley,R.G., and Keppler,D. (1994). The MRP gene encodes an ATP-dependent export pump for leukotriene C4 and structurally related conjugates. J. Biol. Chem. 269, 27807-27810.
43. Luciani,M.F., Denizot,F., Savary,S., Mattei,M.G., and Chimini,G. (1994). Cloning of two novel ABC transporters mapping on human chromosome 9. Genomics 21, 150-159.
44. Madon,J., Hagenbuch,B., Landmann,L., Meier,P.J., and Stieger,B. (2000). Transport function and hepatocellular localization of mrp6 in rat liver. Mol. Pharmacol. 57, 634-641.
45. Matsumura,Y., Umekawa,T., Kawamura,H., Takaoka,M., Robinson,P.S., Cook,N.D., and Morimoto,S. (1992). A simple method for measurement of phosphoramidon-sensitive endothelin converting enzyme activity. Life Sci. 51, 1603-1611.
46. Mayer,R., Kartenbeck,J., Buchler,M., Jedlitschky,G., Leier,l., and Keppler,D. (1995). Expression of the MRP gene-encoded conjugate export pump in liver and its selective absence from the canalicular membrane in transport-deficient mutant hepatocytes. J. Cell Biol. 131, 137-150.
47. Meier,P.J., and Boyer,J.L. (1990). Preparation of basolateral (sinusoidal) and canalicular plasma membrane vesicles for the study of hepatic transport processes. Methods Enzymol. 192, 534-545.
48. Noe,J., Stieger,B., and Meier,P.J. (2002). Functional expression of the canalicular bile salt export pump of human liver. Gastroenterology 123, 1659-1666.
49. Pavek,P., Merino,G., Wagenaar,E., Bolscher,E., Novotna,M., Jonker,J.W., and SchinkeI,A.H. (2005). Human breast cancer resistance protein: interactions with steroid drugs, hormones, the dietary carcinogen 2-amino-1-methyl-6-phenylimidazo(4,5-b)pyridine, and transport of cimetidine. J. Pharmacol. Exp. Ther. 312, 144-152.
50. Pratt,S., Shepard,R.L., Kandasamy,R.A., Johnston,P.A., Perry,W., III, and Dantzig,A.H. (2005). The multidrug resistance protein 5 (ABCC5) confers resistance to 5-fluorouracil and transports its monophosphorylated metabolites. Mol. Cancer Ther. 4, 855-863.
51. Raggers,R.J., van,H.A., Evers,R., and van,M.G. (1999). The human multidrug resistance protein MRP1 translocates sphingolipid analogs across the plasma membrane. J. Cell Sci. 112 (Pt 3), 415-422.
52. Raggers,R.J., Vogels,l., and van,M.G. (2001). Multidrug-resistance P-glycoprotein (MDR1) secretes platelet-activating factor. Biochem. J. 357, 859-865.
53. Reid,G., Wielinga,P., Zelcer,N., van,d.H., I, Kuil,A., de Haas,M., Wijnholds,J., and Borst,P. (2003). The human multidrug resistance protein MRP4 functions as a prostaglandin efflux transporter and is inhibited by nonsteroidal antiinflammatory drugs. Proc. Natl. Acad. Sci. U. S. A 100, 9244-9249.
54. Ringpfeil,F., Lebwohl,M.G., Christiano,A.M., and Uitto,J. (2000). Pseudoxanthoma elasticum: mutations in the MRP6 gene encoding a transmembrane ATP-binding cassette (ABC) transporter. Proc. Natl. Acad. Sci. U. S. A 97, 6001-6006.
55. Riordan,J.R., Rommens,J.M., Kerem,B., Alon,N., Rozmahel,R., Grzelczak,Z., Zielenski,J., Lok,S., Plavsic,N., Chou,J.L., and. (1989). Identification of the cystic fibrosis gene: cloning and characterization of complementary DNA. Science 245, 1066-1073.
56. Rius,M., Nies,A.T., Hummel-Eisenbeiss,J., Jedlitschky,G., and Keppler,D. (2003). Cotransport of reduced glutathione with bile salts by MRP4 (ABCC4) localized to the basolateral hepatocyte membrane. Hepatology 38, 374-384.
57. Rius,M., Thon,W.F., KeppIer,D., and Nies,A.T. (2005). Prostanoid transport by multidrug resistance protein 4 (MRP4/ABCC4) localized in tissues of the human urogenital tract. J. Urol. 174, 2409-2414.
58. Sarkadi,B., Price,E.M., Boucher,R.C., Germann,U.A., and Scarborough,G.A. (1992). Expression of the human multidrug resistance cDNA in insect cells generates a high activity drug-stimulated membrane ATPase. J. Biol. Chem. 267, 4854-4858.
59. Strautnieks,S.S., Bull,L.N., Knisely,A.S., Kocoshis,S.A., Dahl,N., Arnell,H., Sokal,E., Dahan,K., Childs,S., Ling,V., Tanner,M.S., Kagalwalla,A.F., Nemeth,A., Pawlowska,J., Baker,A., Mieli-Vergani,G., Freimer,N.B., Gardiner,R.M., and Thompson,R.J. (1998). A gene encoding a liver-specific ABC transporter is mutated in progressive familial intrahepatic cholestasis. Nat. Genet. 20, 233-238.
60. Sun,H., Molday,R.S., and Nathans,J. (1999). Retinal stimulates ATP hydrolysis by purified and reconstituted ABCR, the photoreceptor-specific ATP-binding cassette transporter responsible for Stargardt disease. J. Biol. Chem. 274, 8269-8281.
61. Takeuchi,T., Yoshitomi,S., Higuchi,T., Ikemoto,K., Niwa,S., Ebihara,T., Katoh,M., Yokoi,T., and Asahi,S. (2006). Establishment and characterization of the transformants stably-expressing MDR1 derived from various animal species in LLC-PK1. Pharm. Res. 23, 1460-1472.
62. Tammur,J., Prades,C., Arnould,l., Rzhetsky,A., Hutchinson,A., Adachi,M., Schuetz,J.D., Swoboda,K.J., Ptacek,L.J., Rosier,M., Dean,M., and Allikmets,R. (2001). Two new genes from the human ATP-binding cassette transporter superfamily, ABCC11 and ABCC12, tandemly duplicated on chromosome 16q12. Gene 273, 89-96.
63. Tanigawara,Y., Okamura,N., Hirai,M., Yasuhara,M., Ueda,K., Kioka,N., Komano,T., and Hori,R. (1992). Transport of digoxin by human P-glycoprotein expressed in a porcine kidney epithelial cell line (LLC-PK1). J. Pharmacol. Exp. Ther. 263, 840-845.
64. Trowsdale,J., Hanson,l., Mockridge,I., Beck,S., Townsend,A., and Kelly,A. (1990). Sequences encoded in the class II region of the MHC related to the 'ABC' superfamily of transporters. Nature 348, 741-744.
65. van AubeI,R.A., Smeets,P.H., Peters,J.G., Bindels,R.J., and Russel,F.G. (2002). The MRP4/ABCC4 gene encodes a novel apical organic anion transporter in human kidney proximal tubules: putative efflux pump for urinary cAMP and cGMP. J Am. Soc. Nephrol. 13, 595-603.
66. Van der Bliek,A.M., Baas,F., Ten Houte,d.L., Kooiman,P.M., Van,d., V, and Borst,P. (1987). The human mdr3 gene encodes a novel P-glycoprotein homologue and gives rise to alternatively spliced mRNAs in liver. EMBO J. 6, 3325-3331.
67. Volk,E.L., and Schneider,E. (2003). Wild-type breast cancer resistance protein (BCRP/ABCG2) is a methotrexate polyglutamate transporter. Cancer Res. 63, 5538-5543.
68. Wang,J., Sun,F., Zhang,D.W., Ma,Y., Xu,F., Belani,J.D., Cohen,J.C., Hobbs,H.H., and Xie,X.S. (2006). Sterol transfer by ABCG5 and ABCG8: In vitro assay and reconstitution. J. Biol. Chem.
69. Wielinga,P., Hooijberg,J.H., Gunnarsdottir,S., Kathmann,l., Reid,G., Zelcer,N., van der,B.K., de,H.M., van,d.H., I, Kaspers,G., Wijnholds,J., Jansen,G., Peters,G., and Borst,P. (2005). The human multidrug resistance protein MRP5 transports folates and can mediate cellular resistance against antifolates. Cancer Res. 65, 4425-4430.
70. Young,S.G., and Fielding,C.J. (1999). The ABCs of cholesterol efflux. Nat. Genet. 22, 316-318.
71. Zelcer,N., Reid,G., Wielinga,P., Kuil,A., van,d.H., I, Schuetz,J.D., and Borst,P. (2003). Steroid and bile acid conjugates are substrates of human multidrug-resistance protein (MRP) 4 (ATP-binding cassette C4). Biochem. J 371, 361-367.
72. Zeng,H., Liu,G., Rea,P.A., and Kruh,G.D. (2000). Transport of amphipathic anions by human multidrug resistance protein 3. Cancer Res. 60, 4779-4784.

## Claims

1. A method for determining transport activity of a transport protein, **characterized in that** the method comprises:
- mixing of
(i) a vesicle which harbours at least one transport protein in a way that the very C-terminus of the transport protein is outside of the vesicle with
(ii) a SPA bead suitable for direct or indirect binding with the very C-terminus of the transport protein with
(iii) - only in case of indirect binding of a SPA bead with the very C-terminus of the transport protein - at least one molecule which can mediate binding of the SPA bead with the very C-terminus of the transport protein with
(iv) a radioactively marked substrate which can be transported by the transport protein under condition which allow transport of the substrate, and
- incubating the mixture for a time period sufficiently long to enable direct or indirect binding of the vesicles with the SPA bead and to enable substrate transport into the vesicle, and
- measuring light emitted by scintillant of the bead.

2. A method according to claim 1, wherein the vesicle is an inside-out vesicle.

3. A method according to claim 1 or 2 wherein the transport protein is an ABC-transport protein.

4. A method according to claim 3, wherein the transport protein is selected from a group consisting of MRP1 (ABCC1), MRP2 (ABCC2), MRP3 (ABCC3), MRP4 (ABCC4), MRP5 (ABCC5), MRP6 (ABCC6), MRP7 (ABCC10), MRP8 (ABCC11), SUR1 (ABCC8), SUR2 (ABCC9), CFTR (ABCC7), ABCA1, ABCA3, ABCA4, ABCG5, ABCG8, MDR1 (ABCB1), MDR3 (ABCB4), BSEP (ABCB11), BCRP (ABCG2), TAP1 (ABCB2), and TAP2 (ABCB3).

5. A method according to any one of claims 1-4, wherein the substrate is selected from a group consisting of ADP, BQ-123, cholesterol, cimetidine, colchicine, cyclic AMP, cyclic GMP, dehydroepiandrosterone sulfate, daunomycin, digoxin, estradiol glucuronide, estrone 3-sulfate, etoposide, folic acid, glucosylceramide, glycochenodeoxycholate, glycocholate, leukotriene C4, leukotriene D4, leukotriene E4, methotrexate, mitoxanthone, sulfobromophthalein, paclitaxel, platelet activating factor, prostaglandin E1, prostaglandin E2, prostaglandin F2alpha, ritonavir, saquinavir thromboxane B2, verapamil, sitosterol, taurochenodeoxycholate, taurocholate, tauroursodeoxycholate, prazosin, vincristine and vinblastine.

6. A method according to any one of claims 1-4 wherein as a transport protein and as a substrate one of the following combinations is used:
| transport protein /gene symbol : | substrate |
|---|---|
| MRP1/ABCC1 : | leukotriene C4, leukotriene D4, leukotriene E4, estradiol glucuronide, folic acid, or methotrexate; |
| MRP2 / ABCC2 : | leukotriene C4, estradiol glucuronide, folic acid, methotrexate, or sulfobromophthalein; |
| MRP3 / ABCC3 : | leukotriene C4, estradiol glucuronide, folic acid, methotrexate, or glycocholate; |
| MRP4 / ABCC4 : | leukotriene C4, estradiol glucuronide, estrone 3-sulfate, dehydroepiandrosterone sulphate, folic acid, cGMP, cAMP, ADP, methotrexate, Prostaglandin E1, prostaglandin E2, prostaglandin F2alpha, or thromboxane B2; |
| MRP5 / ABCC5 : | folic acid, cGMP, cAMP, methotrexate, 5-fluoro-2'-deoxyuridine 5'-monophosphate, 5-fluoro-uridine 5'-monophosphate,or 2'-deoxyuridine 5'-monophosphate ; |
| MRP6 / ABCC6 : | leukotriene C4, or BQ-123; |
| CFTR / ABCC7 : | chloride; |
| MRP7/ABCC10 : | leukotriene C4, or estradiol glucuronide; |
| MRP8 / ABCC11 : | cAMP, cGMP, leukotriene C4, estradiol glucuronide, estrone 3-sulfate, dehydroepiandrosterone sulphate, taurocholate, or glycocholate; |
| MDR1/ABCB1 : | glucosylceramide, platelet activating factor, daunomycin, digoxin, colchicine, etoposide, paclitaxel, verapamil, vincristine, vinblastine, ritonavir, or saquinavir; |
| BSEP/ABCB11 : | tauroholate, glycocholate, taurochenodeoxycholate, glycochenodeoxycholate, or tauroursodeoxycholate: |
| ABCA4 : | retinal; |
| BCRP / ABCG2 : | estrone 3-sulfate, estradiol glucuronide, folic acid, methotrexate, mitoxanthone, topotecan, or cimetidine; |
| ABCG5 : | cholesterol, or sitosterol; |
| ABCG8 : | cholesterol, or sitosterol. |

7. A method according to any one of claims 1-6, wherein the incubation period lasts at least for 30 seconds and lasts no longer than 30 hours.

8. A method according to claim 7, wherein the incubation period lasts at least for 1 hour and lasts no longer than 24 hours.

9. A method for measuring transport activity of a transport protein in an high throughput (HTS) format **characterized in that** a method according to any one of claim 1-8 is performed.

10. A method according to claim 9 in which a plate having at least 96 wells is used.

11. A method according to claim 9 in which a 384 well plate or a 1536 well plate is used.

12. A method according to claim 9 in which a chip is used as reaction and/or readout platform.

13. A method according to any one of claims 1-12 in which as a transport protein MRP4 is used.

14. A method according to claim 13 in which in (iii) of the method of claim 1 an antibody which can bind to a peptide which sequence is given in SEQ ID NO 1 or SEQ ID NO 2 is used.

15. A method according to claim 13 or 14 in which in (ii) of the method of claim 1 a SPA bead which has been coated with Protein A is used.

16. A method according to claim 13 or 14 in which in (ii) of the method of claim 1 a SPA bead which has been coated with a further antibody which can bind to the antibody according to claim 14 is used.

17. A method according to any one of claims 1-12 in which as a transport protein MRP4 to which a histidine tag has been C-terminally fused is used.

18. A method according to claim 17 in which as a transport protein MRP4 to which a histidine tag consisting of at least 6 histidine residues is used.

19. A method according to claim 17 or 18 in which in (ii) of the method of claim 1 a SPA bead which has been coated with copper-chelate is used.

20. A method for determining whether a compound is a modulator of transport activity of a transport protein, **characterized in that** the method comprises:
- mixing of
(A)
(i) a vesicle which harbours at least one transport protein in a way that the very C-terminus of the transport protein is outside of the vesicle with
(ii) a SPA bead suitable for direct or indirect binding with the very C-terminus of the transport protein with
(iii) - only in case of indirect binding of a SPA bead with the very C-terminus of the transport protein - at least one molecule which can mediate binding of the SPA bead with the very C-terminus of the transport protein with
(iv) a radioactively marked substrate which can be transported by the transport protein under condition which allow transport of the substrate with
(v) a compound to be tested, and mixing of
(B)
(i) a vesicle which harbours at least one transport protein in a way that the very C-terminus of the transport protein is outside of the vesicle with
(ii) a SPA bead suitable for direct or indirect binding with the very C-terminus of the transport protein with
(iii) - only in case of indirect binding of a SPA bead with the very C-terminus of the transport protein - at least one molecule which can mediate binding of the SPA bead with the very C-terminus of the transport protein with
(iv) a radioactively marked substrate which can be transported by the transport protein under condition which allow transport of the substrate with, and
- incubating the mixture (A) and (B) for the same time period sufficiently long to enable direct or indirect binding of the vesicles with the SPA bead and to enable substrate transport into the vesicle, and
- measuring light emitted by scintillant of the bead, wherein increased value obtained with (A), when compared with (B), identifies a compound which is a activator of the transport protein tested and a decreased value obtained with (A), when compared with(B), identifies a compound which is an inhibitor of the transport protein tested.

21. A method according to claim 20, wherein the vesicle is an inside-out vesicle.

22. A method according to claim 20 or 21 wherein the transport protein is an ABC-transport protein.

23. A method according to claim 22, wherein the transport protein is selected from a group consisting of: MRP1 (ABCC1), MRP2 (ABCC2), MRP3 (ABCC3), MRP4 (ABCC4), MRP5 (ABCC5), MRP6 (ABCC6), MRP7 (ABCC10), MRP8 (ABCC11), SUR1 (ABCC8), SUR2 (ABCC9), CFTR (ABCC7), ABCA1, ABCA3, ABCA4, ABCG5, ABCG8, MDR1 (ABCB1), MDR3 (ABCB4), BSEP (ABCB11), BCRP (ABCG2), TAP1 (ABCB2), and TAP2 (ABCB3)..

24. A method according to any one of claims 20-23, wherein the substrate is selected from a group consisting of: ADP, BQ-123, cholesterol, cimetidine, colchicine, cyclic AMP, cyclic GMP, dehydroepiandrosterone sulfate, daunomycin, digoxin, estradiol glucuronide, estrone 3-sulfate, etoposide, folic acid, glucosylceramide, glycochenodeoxycholate, glycocholate, leukotriene C4, leukotriene D4, leukotriene E4, methotrexate, mitoxanthone, sulfobromophthalein, paclitaxel, platelet activating factor, prostaglandin E1, prostaglandin E2, prostaglandin F2alpha, ritonavir, saquinavir thromboxane B2, verapamil, sitosterol, taurochenodeoxycholate, taurocholate, tauroursodeoxycholate, prazosin, vincristine and vinblastine.

25. A method according to any one of claims 20-23 wherein as a transport protein and as a substrate one of the following combinations is used:
| transport protein / gene symbol : | substrate |
|---|---|
| MRP1/ABCC1 : | leukotriene C4, leukotriene D4, leukotriene E4, estradiol glucuronide, folic acid, or methotrexate; |
| MRP2 / ABCC2 : | leukotriene C4, estradiol glucuronide, folic acid, methotrexate, or sulfobromophthalein; |
| MRP3 / ABCC3 : | leukotriene C4, estradiol glucuronide, folic acid, methotrexate, or glycocholate; |
| MRP4 / ABCC4 : | leukotriene C4, estradiol glucuronide, estrone 3-sulfate, dehydroepiandrosterone sulphate, folic acid, cGMP, cAMP, ADP, methotrexate, Prostaglandin E1, prostaglandin E2, prostaglandin F2alpha, or thromboxane B2; |
| MRP5 / ABCC5 : | folic acid, cGMP, cAMP, methotrexate, 5-fluoro-2'-deoxyuridine 5'-monophosphate, 5-fluoro-uridine 5'-monophosphate,or 2'-deoxyuridine 5'-monophosphate ; |
| MRP6 / ABCC6 : | leukotriene C4, or BQ-123; |
| CFTR / ABCC7 : | chloride; |
| MRP7/ABCC10 : | leukotriene C4, or estradiol glucuronide; |
| MRP8 / ABCC11 : | cAMP, cGMP, leukotriene C4, estradiol glucuronide, estrone 3-sulfate, dehydroepiandrosterone sulphate, taurocholate, or glycocholate; |
| MDR1/ABCB1 : | glucosylceramide, platelet activating factor, daunomycin, digoxin, colchicine, etoposide, paclitaxel, verapamil, vincristine, vinblastine, ritonavir, or saquinavir; |
| BSEP / ABCB11 : | tauroholate, glycocholate, taurochenodeoxycholate, glycochenodeoxycholate, or tauroursodeoxycholate: |
| ABCA4 : | retinal; |
| BCRP / ABCG2 : | estrone 3-sulfate, estradiol glucuronide, folic acid, methotrexate, mitoxanthone, topotecan, or cimetidine; |
| ABCG5 : | cholesterol, or sitosterol; |
| ABCG8 : | cholesterol, or sitosterol. |

26. A method according to any one of claims 20-25, wherein the incubation period lasts at least for 30 seconds and lasts no longer than 30 hours.

27. A method according to claim 26, wherein the incubation period lasts at least for 1 hour and lasts no longer than 24 hours.

28. A method for measuring transport activity of a transport protein in an high throughput (HTS) format **characterized in that** a method according to any one of claim 20-27 is performed.

29. A method according to claim 28 in which a plate having at least 96 wells is used.

30. A method according to claim 28 in which a 384 well plate or a 1536 well plate is used.

31. A method according to claim 28 in which a chip is used as reaction and/or readout platform.

32. A method according to any one of claims 20-31 in which as a transport protein MRP4 is used.

33. A method according to claim 32 in which in (iii) of the method of claim 20 an antibody which can bind to a peptide which sequence is given in SEQ ID NO 1 or SEQ ID NO 2 is used.

34. A method according to claim 32 or 33 in which in (ii) of the method of claim 20 a SPA bead which has been coated with Protein A is used.

35. A method according to claim 32 or 33 in which in (ii) of the method of claim 20 a SPA bead which has been coated with a further antibody which can bind to the antibody according to claim 33 is used.

36. A method according to any one of claims 20-31 in which as a transport protein MRP4 to which a histidine tag has been C-terminally fused is used.

37. A method according to claims 36 in which as a transport protein MRP4 to which a histidine tag consisting of at least 6 histidine residues is used.

38. A method according to claim 36 or 37 in which in (ii) of the method of claim 20 a SPA bead which has been coated with copper-chelate is used.

39. Use of a method according to any one of claims 1-38 in a high throughput screening or in an ultra high throughput screening.

40. A kit for determining transport activity of a transport protein comprising:
(a) a vesicle which harbours at least one transport protein in a way that the very C-terminus of the transport protein is outside of the vesicle, and
(b) SPA bead suitable for direct or indirect binding with the very C-terminus of the transport protein.


SEQUENCE LISTING

<110> BOEHRINGER INGELHEIM PHARMA GmbH & CO KG

<120> Method for determining transport activity of a transport protein

<130> p01-2128

<160> 2

<170> PatentIn version 3.3

<210> 1
<211> 15
<212> PRT
<213> human

<400> 1

Ser Asn Gly Gln Pro Ser Thr Leu Thr ILe Phe Glu Thr Ala Leu
1               5                   10                  15

<210> 2
<211> 25
<212> PRT
<213> human

<400> 2

Gly His Thr Asp His Met Val Thr Asn Thr Ser Asn Gly Gln Pro Ser Thr Leu Thr ILe
1               5                   10                  15                  20

Phe Glu Thr Ala Leu
21              25